(19)

**Europäisches Patentamt**
**European Patent Office**
**Office européen des brevets**

(11) **EP 2 353 716 A1**

(12) **EUROPEAN PATENT APPLICATION**

(43) Date of publication:
**10.08.2011 Bulletin 2011/32**

(51) Int Cl.:
**B01L 3/00** (2006.01)   **B01L 7/00** (2006.01)
**C12Q 1/68** (2006.01)   **F04B 19/00** (2006.01)

(21) Application number: **10001234.3**

(22) Date of filing: **05.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Ludwig-Maximilians-Universität München**
**80539 München (DE)**

(72) Inventors:
• **Braun, Dieter.**
**80799 München (DE)**
• **Mast, Christof**
**85406 Zolling (DE)**

(74) Representative: **Lucke, Andreas et al**
**Forrester & Boehmert**
**Pettenkoferstrasse 20-22**
**80336 München (DE)**

(54) **Method and apparatus for amplifying nucleic acid sequences**

(57) A method of amplifying one or more target nucleic acid sequence(s) in a PCR buffer solution comprising one or more target nucleic acid sequence(s), a nucleic acid polymerase, NTPs, at least a first primer and a second primer is provided. The PCR buffer solution is contained in an elongated vessel (2). The solution within the vessel (2) is locally heated in a spatio-temporally varying manner. Fluid flows (31-34) circulating within the vessel are established. At least a fraction of the PCR buffer solution cyclically travels between regions (35, 36; 37, 38) having different temperatures, giving rise to a temperature cycling.

Fig. 3A

**(Cont. next page)**

Fig. 3B

**Description**

The invention relates to a method of and an apparatus for amplifying a nucleic acid sequence. The invention relates in particular to a method and an apparatus that allow a nucleic acid sequence to be amplified by polymerase chain reaction (PCR).

The polymerase chain reaction (PCR) has become the conventional technique used to amplify specific DNA or RNA sequences. U.S. 4,683,202 and U.S. 4,683,195 describe the basic PCR technique. Since the first disclosure of the PCR method, it has had a profound effect on the practice of biotechnology and biomedical science. A wide range of applications, such as the replication of DNA specimens in DNA forensics or in medical diagnostics, is based on PCR for replicating nucleic acid sequences.

Generally, the PCR proceeds in three steps which are periodically repeated. These steps are: (i) heating a double stranded (ds) DNA or RNA at a temperature of approximately 95°C to dissociate the double helix into two strands (denaturation or melting); (ii) decreasing the temperature to approximately 50-65°C in the presence of an excess of two primers, which are complementary to the terminal target sequences (i.e., the sequences at 5' and 3' ends) and form short double strands at the ends (annealing); and (iii) raising the temperature to approximately 72°C in the presence of a thermostable polymerization enzyme called nucleic acid polymerase which elongates each of the two short double strands to generate twice the amount of the target DNA (elongation). Frequently, the temperature cycling may be simplified to only two temperature levels as has been demonstrated, for example in Modern Physics Letters B, Vol. 18, No. 16 (2004) 775-784, or Science Vol. 298. no. 5594 (2002), p. 793.

Advances in instrumentation have aided the wide application of PCR. PCR cyclers provide a large degree of automation and thus enhance convenience for the user. However, PCR cyclers that apply a temperature sequence to a reaction vessel may be costly. Further, many conventional PCR cyclers have a large thermal mass, which makes it challenging to realize rapid temperature changes. Realizing rapid temperature changes may be desirable considering that the speed limit of PCR may be as short as 15-30 s for one temperature cycle if PCR is only used to detect a DNA with a certain sequence, in which case the length of the amplified DNA may be selected to be only 50-100 base pairs short.

A major problem with nucleic acid amplification via PCR is the generation of unspecific amplification products. Very often this is due to an unspecific oligonucleotide priming and subsequent primer extension event prior to the actual thermosizing procedure itself. Frequently observed are also amplification products due to primer dimerization and subsequent extension.

Approaches that utilize microfluidic devices, in which PCR buffer solution is driven through microfluidic channels and sequentially passes regions having different temperatures, have also been pursued. A microfluidic device for fast DNA replication by PCR is for example described in US 6,428,987 B2. While such microfluidic chips do not suffer from a large thermal mass which imposes limits on temperature change rates, manufacture of microfluidic chips may be costly. Further, depending on the design of the microfluidic chip, micropumps and/or microvalves may need to be provided to drive the solution through the microfluidic chip, which adds to the costs of the PCR setup.

Recently, approaches to realize PCR have been described in D. Braun, N. L. Goddard and A. Libchaber, "Exponential DNA Replication by Laminar Convection", PRL 91, 138103 (2003) and in M. Krishnan, V. M. Ugaz and M. A. Burns, "PCR in a Rayleigh-Bénard Convection Cell", Science 298, 793 (2002) in which microthermal convection provides the temperature cycling necessary for driving the PCR. In these approaches, a microfluidic column is heated asymmetrically, such that at one side of the column the PCR buffer solution has a temperature that allows the melting step of the PCR to take place, while at another side of the column the PCR buffer solution has temperature that allows the elongation step of the PCR to take place. The different temperatures also give rise to a convective flow in the microfluidic column. These approaches have the advantage that the temperature cycling required for the PCR to take place can be implemented by statically heating one side of the microfluidic column. However, in this approach, the replicated DNA will generally exhibit only little concentration variations in the microfluidic column due to diffusion. In some applications, it may be desirable to have an enhanced concentration of replicated nucleic acid sequences in one or plural regions of a microfluidic vessel, which would facilitate extraction of the replicated nucleic acid sequences from the microfluidic vessel for further use.

P. Baaske et al., "Extreme accumulation of nucleotides in simulated hydrothermal pore systems", PNAS 104 (22), 9346 (2007) describe that the interplay of thermophoresis or thermodiffusion, i.e., the motion of a molecule along a temperature gradient, and convective flow in a column-type geometry may give rise to the accumulation of DNA molecules in certain regions within the column. The width of the column influences which molecule lengths will reach a high concentration in certain regions of the column. It may be challenging to accommodate different vessel widths in an apparatus for replicating nucleic acid sequences by PCR.

F. M. Weinert and D. Braun, "An Optical Conveyor for Molecules", Nano Lett. 2009, 9(12), pp. 4264-4267 describe that a concentration variation in a disc-shape geometry can be attained by combining a bidirectional flow with a perpendicular thermophoretic molecule drift. In a toroid geometry, the hundredfold accumulation of 5-base DNA within seconds has been observed. No PCR has been realized in the disc-shaped geometry.

F. M. Weinert and D. Braun, "Optically driven fluid flow along arbitrary microscale patterns using thermoviscous expansion", J. Appl. Phys. 104, 104701 (2008) describe that fluid can be moved by a laser scanning microscope, due to an asymmetric thermal expansion in a front of a laser spot and the asymmetric thermal contraction in its wake.

There is still a need in the art for an improved method of and an apparatus for replicating amplification nucleic acid sequences. In particular, there is a need in the art for a method of and an apparatus for replicating nucleic acid sequences which do not suffer from the problems associated with the large thermal mass of conventional PCR cyclers and the complexity of some microfluidic chip designs. There is further a need in the art for a method of and an apparatus for replicating nucleic acid sequences which allow nucleic acid sequences to be accumulated in one region or plural regions of a reaction vessel in a controllable manner. There is further a need in the art for a method of and an apparatus for selectively replicating/amplifying a selected target sequence. A method and the apparatus shall be suitable for rapidly and efficiently amplifying a wide variety of nucleic acid sequences.

According to the invention, the above need is addressed by a method of amplifying one or more nucleic acid sequence(s) in a PCR buffer solution and an apparatus for amplifying one or more nucleic acid sequence(s) in a PCR buffer solution as defined by the independent claims. The dependent claims define preferred or advantageous embodiments.

According to an aspect of the invention, a method of amplifying one or more target nucleic acid sequence(s) in a PCR buffer solution which comprises one or more target nucleic acid sequence(s), a nucleic acid polymerase, NTPs, at least a first primer and a second primer, wherein the PCR buffer solution is contained in an elongated vessel is provided. The method comprises establishing a spatially varying temperature in the PCR buffer solution, such that

-    in a first region within the vessel, the PCR buffer solution has a temperature at which a denaturation step of a PCR takes place,
-    in a second region within the vessel, the PCR buffer solution has a temperature at which an annealing step and an elongation step of a PCR takes place, and
-    the first region and the second region are offset relative to each other in a transverse direction of the vessel.

The method further comprises locally supplying heating power to the PCR buffer solution at a plurality of positions disposed along a longitudinal direction of the vessel in a time-varying manner to drive the PCR buffer solution so as to establish a fluid flow circulating within the vessel, such that at least a fraction of the PCR buffer solution cycles between the first region and the second region.

In the method according to the invention, a fluid flow is established in the PCR buffer solution by supplying heating power to the PCR buffer solution at a plurality of positions in a time-varying manner, i.e., by using a spatio-temporally varying heating. This mechanism for driving the fluid flow allows a velocity of the fluid flow within the vessel to be adjusted. It has been found that, surprisingly, by adjusting the velocity of the fluid flow within the vessel, a local concentration increase of molecules or particles comprised by the PCR buffer solution may be controlled. The mechanism is selective in the sense that, for a given velocity of the fluid flow, the obtained concentration changes are more pronounced for one type of molecules, complexes or particles than for other types of molecules, complexes or particles. It is noted that the term "selective" does not preclude that more than one type of molecules, complexes or particles is accumulated in a region of the vessel.

Further surprisingly, the one or several nucleic acid sequence(s) may still be replicated by PCR when the velocity is set such that the PCR buffer solution exhibits a local variation in concentration of the nucleic acid sequence(s).

As used herein, the term "nucleic acid sequence" includes DNA and RNA sequences of variable length and sequence. The term "nucleic acid sequence" further includes single strand (ss) and double strand (ds) nucleic acid sequences.

As used herein, a supplying of heating power is considered to be "local" if heating power is supplied to the solution over an area or in a volume that allows spatial temperature variations to be established in at least one transverse direction of the vessel.

In an embodiment, the locally supplying heating power may be implemented by scanning a beam of electromagnetic radiation, in particular an IR laser beam, over the plurality of positions. The beam of electromagnetic radiation may be focussed such that, on passage through the vessel, the beam diameter is smaller than at least the inner diameter of the vessel in the direction transverse to both the beam direction and the longitudinal direction of the vessel.

In an embodiment, the elongated vessel may be arranged such that a longitudinal axis of the vessel is horizontally arranged.

The PCR buffer solution may be any one of various conventional and commercially available PCR buffer solutions. That PCR buffer solution may be adapted within the scope of the present invention in relation to the specific target molecule(s) to be amplified. For example, the pH value and/or the ionic strength of the buffer solution may be varied. Such variations are well known in the art and may be carried out using common general knowledge. For illustration rather than limitation, the PCR buffer solution may be a commercial Qiagen Fast Cycling Kit. Other conventional PCR buffer solutions may also be used. Examples for such PCR buffer solutions are given in Cornel Mülhardt, "Der Experimentator: Molekularbiologie/Genomics", Spektrum Akademischer Verlag, 6. Aufl. (2008).

The DNA polymerase to be used within the scope of the present invention may be any enzyme which is capable of performing a template-dependent primer extension reaction. Such DNA polymerase enzymes are commercially available and may be chosen in accordance with the intended reaction. Examples of such DNA polymerases are RNA template-dependent polymerase, DNA template-dependent polymerase, DNA-dependent DNA polymerase with additional RNA template-dependent reverse transcriptase activity. Examples of DNA polymerases to be used according to the invention are the Taq DNA polymerase or the thermostable DNA polymerases from *Thermus thermophilus.*

The primers to be used are primer oligonucleotides which are completely or almost completely complementary to a specific region of the target nucleic acid. For specific purposes such a primer may be chemically modified, for example by attaching at its 3' end a fluorescent label. The construction of primers as well as their modification are well known to a person skilled in the art.

If the PCR is to be conducted as a real time PCR, additionally included in the PCR solution is a double-stranded DNA binding dye component such as SYBR green or any other fluorescently labelled hybridization probes such as TaqMan probes, molecular beacons, FRET hybridization probes or simple probes.

If the PCR is to be carried out as an asymmetric PCR (aPCR) by tweaking primer concentrations, different concentrations of the two primers (upstream and downstream primers) are used. As the cycles increase primers with a lower concentration are used up while primers with a high concentration continue to produce single-chain DNA at a linearly increasing rate. It is also possible to use other variants of asymmetric PCR, for example such as the use of different lengths of upstream and downstream primers. In this case, during the second phase of the PCR cycles the annealing temperature has to be increased. The short primers are unable to anneal under such conditions while the long primers continue to extend to obtain a single-chain nucleic acid. It should be possible to reduce laserpower after the first phase of aPCR and simultaneously to adapt the laserpattern to maintain the current fluid velocity. Therefore, the thermal gradient is reduced while the base temperature given by the peltier elements has to be increased at the same time. This leads to a higher annealing/elongation temperature (needed for this type of aPCR) while all other parameters of the experiment are kept constant. The PCR may also be carried out as an asynchronous PCR by tweaking annealing temperatures.

The nucleic acid sequence(s) to be replicated does not need to be comprised by the PCR buffer solution which is initially provided to start the PCR reaction. Rather, the PCR buffer solution may be a mutagenic PCR buffer solution, and the nucleic acid sequence(s) which is or are to be replicated may be generated by mutation on the basis of a given nucleic acid sequence in the solution.

In the method, a fluid flow circulating within the vessel is established. In a solution, diffusion processes will be present in addition to a circulating fluid flow, which may cause molecules to diffuse into and out of the fraction of the PCR buffer solution which cyclically travels between the first and second regions, respectively.

In the method, at least one of heating energy respectively supplied at the plurality of positions and a frequency at which heating energy is respectively supplied at the plurality of positions may be controlled to set a velocity of the PCR buffer solution at the plurality of positions. By setting the velocity of the fluid flow in the PCR buffer solution, a thermodynamic equilibrium condition may be established in which a desired molecule, complex or particle exhibits more pronounced variations in local concentration than other molecules, complexes or particles which are also comprised by the PCR buffer solution.

In the method, the locally supplying heating power may comprise scanning a beam of electromagnetic radiation, in particular an IR laser beam, in a first direction along a first line segment which extends along a longitudinal direction of the vessel, and scanning the beam of electromagnetic radiation in a second direction along a second line segment which extends along the longitudinal direction of the vessel, the second direction being opposite to the first direction. Thereby, counter-rotating bi-directional flows may be established along the vessel longitudinal direction. This allows an accumulation of molecules, complexes or particles to be established in a region of the vessel which is spaced from its longitudinal ends. For a vessel having open ends, an unintentional escape of the molecules, complexes or particles which are to be accumulated through the open ends may be reduced.

In the method, when a beam of electromagnetic radiation is scanned along the vessel, a power supplied to the PCR buffer solution may be varied as a function of the position at which power is supplied. Thereby, more complex flow patterns comprising one or more circulating flows may be established

In the method according to any one of the above embodiments, the vessel may have a rectangular cross-section, and the heating power may be supplied such that the plurality of positions are spaced from at least two lateral walls of the vessel. In an exemplary embodiment, the heating power may be locally supplied least along a longitudinal centreline of the vessel. By supplying the heating power at positions spaced from at least two lateral walls of the vessel, an uncontrolled diffusion of molecules or particles away from the accumulation region and along one of the lateral walls may be reduced.

In the method of any one of the above embodiments, the heating power may be supplied such that a velocity of the PCR buffer solution at at least some of the plurality of positions is set in dependence on characteristics, in particular in dependence on a diffusion coefficient, of a nucleic acid sequence to be replicated. Thereby, a pronounced local concentration increase of the nucleic acid sequence to be replicated can be attained in specific regions with the vessel.

The heating power may be supplied such that a velocity of the PCR buffer solution is set in dependence on a quotient of the diffusion coefficient of the nucleic acid sequence to be replicated and a transverse width of at least one fluid flow circulating within the vessel. The transverse width of a circulating fluid flow will usually be determined by the plurality of positions at which heating power is locally supplied. In embodiments, the width(s) of the circulating fluid flows may correspond to a distance of the plurality of positions at which heating power is locally supplied from the side walls of the vessel. If the local heating power is supplied by a beam of electromagnetic radiation, the width(s) of at least one convectional fluid flow, in the direction transverse to the vessel longitudinal axis and tangential to the fluid flow at the fluid inversion points, may be equal to a distance or to distances between the plane in which the beam of electromagnetic radiation is scanned and the lateral inner side walls of the vessel.

The heating power may be supplied such the following condition is fulfilled:

$$3,99 \cdot \frac{D}{a} \leq v \leq 5,39 \cdot \frac{D}{a} \ , \qquad (1)$$

where D denotes the diffusion coefficient of the nucleic acid sequence to be replicated, a denotes the transverse width of at least one convectional fluid flow, and v denotes a maximum vessel-averaged velocity which may be controlled by controlling the spatio-temporally varying pattern in which heating power is supplied. In an embodiment, the maximum vessel-averaged velocity may be defined by $v = 2\,v_{meas}/3$, where $v_{meas}$ is the maximum velocity in the three-dimensional vessel. A velocity fulfilling Equation (1) provides an efficient accumulation of various kinds of replicated nucleic acid sequences. Even more preferably, the heating power may be supplied such that the following condition is fulfilled:

$$4,45 \cdot \frac{D}{a} \leq v \leq 4,92 \cdot \frac{D}{a} \ . \qquad (2)$$

In an embodiment, the locally heating may be adjusted such that $v \approx 4,69 \cdot D/a$, with the definitions of v, D and a given above.

In the method, the target nucleic acid sequences may be ssDNA or ssRNA sequences comprising random sequences comprising respectively at their 5' and 3' ends sequences complementary to the primers to be used to allow for PCR amplification. The PCR solution may be a mutagenic PCR solution and may additionally comprise a plurality of target sequences immobilised on a suitable carrier. This allows the method to be utilized for synthesising aptamers. The carrier may be a molecule. In another embodiment, the carrier may be a nano-particle.

The latter embodiment allows the creation of aptamers which bind to the corresponding target sequence, which is immobilized on a suitable carrier. In this case, the PCR solution contains a pool of ssDNA sequences (about $10^{12}$ to $10^{16}$ types of ssDNA) having different sequences, whereby the primer binding sites are conserved among all types of sequences. The PCR reaction is preferably carried out as an asymmetric/asynchronous PCR reaction with a suitably chosen ratio between the first and the second primer. Conveniently, the ratio between the two primers may be between 1:10 and 1:30. Preferably, the ratio between the two primers is 1:20. After a short depletion phase, only single-stranded DNA sequences are created. The use of a mutagenic PCR solution allows the generation of additional mutations in the sequence, thus allowing the creation of new aptamers. In an embodiment of said variant the PCR solution comprises a ssDNA pool comprising binding a non-binding ssDNA molecule in relation to the target sequence. When the reaction is started, molecules binding to the target sequences immobilised on the carrier accumulate in the centre of the vessel and will be discharged for further cloning and sequences. Molecules not binding to the immobilised targets remain in the solution and travel into the region of the asymmetric/asynchronous PCR. They will be amplified and, optionally, mutated so that, optionally, in a further circle of the PCR they may bind to the target molecule on the carrier, thus again accumulating in the centre of the vessel. This means that, as the reaction progresses, molecules binding to an immobilized target sequence (either initially present or *de novo* created by mutation) will selectively enrich, thus allowing the amplification in amounts suitable for further use. By this method, aptamers binding to target sequences not yet fully identified may conveniently be created.

In the method, when the nucleic acid sequences are ssDNA or ssRNA sequences comprising random sequences comprising respectively at their 5' and 3' ends fixed sequences for PCR amplification, and when the PCR solution is a mutagenic PCR solution and additionally comprises a plurality of target sequences immobilised on a carrier, the heating power may be supplied such that a velocity of the PCR buffer solution at the plurality of positions is set in dependence on a diffusion coefficient of the complex between the amplified nucleic acid sequence and the specific target sequence immobilised on the carrier. Thereby, a pronounced local concentration increase of the carrier with the complex immobilised thereon is attainable in specific regions within the vessel.

In the method, when the nucleic acid sequences are ssDNA or ssRNA sequences comprising random sequences comprising respectively at their 5' and 3' ends fixed sequences for PCR amplification, and when the PCR solution is a mutagenic PCR solution and additionally comprises a plurality of target sequences immobilised on a carrier, the heating power may be supplied such that a velocity of the PCR buffer solution at at least some positions of the plurality of positions is set in dependence on a quotient of the diffusion coefficient of the complex between the amplified nucleic acid sequence and the specific target sequence immobilised on the carrier and the transverse width of the vessel. The heating power may be supplied such the following condition is fulfilled:

$$3,99 \cdot \frac{D_{carrier-complex}}{a} \leq v \leq 5,39 \cdot \frac{D_{carrier-complex}}{a} \quad , \qquad (3)$$

where $D_{carrier-complex}$ denotes the diffusion coefficient of the carrier with a complex between the amplified nucleic acid sequence and the specific target sequence immobilised on the carrier, a denotes the transverse width of at least one convectional fluid flow, in the direction transverse to the longitudinal axis of the vessel and tangential to the fluid flow at the fluid inversion points, and v denotes a maximum vessel-averaged velocity which may be controlled by controlling the spatio-temporally varying pattern in which heating power is supplied. In an embodiment, the maximum vessel-averaged velocity may be defined by v = 2 · $v_{meas}$/3, where $v_{meas}$ is the actual maximum velocity within the vessel. A velocity fulfilling Equation (3) provides an efficient accumulation of various kinds of complexes. Even more preferably, the heating power may be supplied such that the following condition is fulfilled:

$$4,45 \cdot \frac{D_{carrier-complex}}{a} \leq v \leq 4,92 \cdot \frac{D_{carrier-complex}}{a} \quad . \qquad (4)$$

In an embodiment, the locally heating may be adjusted such that v≈4,69·D/a, with the definitions of v, D and a given above.

In the method of any one of the above embodiments, the vessel may be open at its longitudinal ends, and the method may comprise establishing a net flow of the PCR buffer solution through the vessel. Thereby, new polymerase molecules and NTPs may be supplied, and the PCR may be performed in a quasi-continuous manner. Further, undesired primer-dimers may be removed from the vessel. A flow velocity of the net flow is advantageously selected such that it is smaller, in particular much smaller, than a maximum velocity of the circulating fluid flow established by locally supplying heating power.

In the method of any one of the above embodiments, the locally supplying heating power to the PCR buffer solution may be performed such that the fluid flow circulating within the vessel is a laminar flow. The locally supplying heating power to the PCR buffer solution may be performed such that a product of the maximum velocity of the circulating fluid flow and the width of the vessel divided by the viscosity of the PCR buffer solution is much smaller than 2000. Thereby, turbulent flows that could reduce the PCR efficiency may be suppressed.

According to another aspect of the invention, an apparatus for amplifying one or more nucleic acid sequence(s) in a PCR buffer solution comprising one or more nucleic acid sequence(s), a nucleic acid polymerase, NTPs, at least a first primer and a second primer, wherein the PCR buffer solution is contained in an elongated vessel is provided. The apparatus comprises support means for supporting the vessel. The apparatus further comprises power supply means configured to locally supply heating power to the PCR buffer solution at a plurality of positions disposed along a longitudinal direction of the vessel in a time-varying manner to drive the PCR buffer solution so as to establish a fluid flow circulating within the vessel and so as to establish a temperature gradient within the vessel. The apparatus further comprises temperature adjustment means configured to adjust at least an offset temperature of the PCR buffer solution, such that

- in a first region within the vessel, the PCR buffer solution has a temperature at which a denaturation step of a PCR takes place,
- in a second region within the vessel, the PCR buffer solution has a temperature at which an annealing step and an elongation step of a PCR takes place, and
- the first region and the second region are offset relative to each other in a transverse direction of the vessel.

The power supply means is configured to locally supply heating power to the PCR buffer solution such that at least a fraction of the solution travels between the first region and the second region.

In this apparatus, the mechanism for driving the fluid flow allows a velocity of the fluid flow within the vessel to be adjusted. It has been found that, surprisingly, by adjusting the velocity of the fluid flow within the vessel, a local concentration increase of molecules, complexes or particles comprised by the PCR buffer solution may be controlled. The mechanism is selective in the sense that, for a given velocity of the fluid flow, the obtained concentration changes are more pronounced for one type of molecules, complexes or particles than for other types of molecules, complexes or particles. It is noted that the term "selective" does not preclude that more than one type of molecules or particles is accumulated in a region of the vessel. Further surprisingly, when using this apparatus, the one or several nucleic acid sequence(s) may still be replicated by PCR when the velocity is set such that the PCR buffer solution exhibits a local variation in concentration of the nucleic acid sequence(s).

Reference is also made to the explanations given for the method according to the invention, in particular as regards term "nucleic acid sequence(s)".

The apparatus may further comprise control means configured to control at least one of heating power respectively supplied by the power supply means at the plurality of positions and a frequency at which the power supply means supplies heating power at the plurality of positions. The control means allows the velocity of the fluid flow in the PCR buffer solution to be adjusted. Thereby, a thermodynamic equilibrium condition may be established in which a desired molecule, complex or particle exhibits more pronounced local concentration variations in the PCR buffer solutions than other molecules or particles which are also comprised by the PCR buffer solution.

When the apparatus comprises a control means, the control means may be configured to control the power supply means such that a velocity of the PCR buffer solution at the plurality of positions is set in dependence on characteristics, in particular in dependence on a diffusion coefficient, of a nucleic acid sequence to be replicated. Thereby, a pronounced local concentration increase of the nucleic acid sequence to be replicated is attainable in specific regions with the vessel.

When the nucleic acid sequences are ssDNA or ssRNA sequences comprising random sequences comprising respectively at their 5' and 3' ends fixed sequences for PCR amplification, and when the PCR solution is a mutagenic PCR solution and additionally comprises a plurality of target sequences immobilised on a carrier, and when the apparatus comprises a control means, the control means may be configured to control the power supply means such that a velocity of the PCR buffer solution at the plurality of positions is set in dependence on a diffusion coefficient of a complex between the amplified nucleic acid sequence and the specific target sequence immobilised on the carrier. Thereby, a pronounced local concentration increase of the carrier with the complex immobilised thereon is attainable in specific regions with the vessel.

In any one of the above embodiments, when the apparatus comprises a control means, the control means may be adapted to control the spatio-temporal pattern in which heating power is locally such that at least one of the criteria described in connection with the methods according to various embodiments is fulfilled.

The apparatus may comprise the vessel supported by the support means.

The apparatus may be configured to perform the method of any one aspect or embodiment described herein.

The methods and apparatuses according to various embodiments may be employed in any one of the various applications in which the replication of nucleic acid sequence(s) is desired. The methods and apparatuses according to various embodiments may in particular be employed in the synthesis and selection of aptamers.

Embodiments of the invention will be described in detail below with reference to the accompanying drawings, wherein:

Fig. 1 is a schematic view of an apparatus according to an embodiment.

Fig. 2 is a schematic view of a vessel for illustrating a spatio-temporal pattern in which heating power is supplied in an embodiment.

Fig. 3A is a schematic diagram illustrating a fluid flow established in the vessel of Fig. 2 according to an embodiment.

Fig. 3B is a schematic diagram illustrating regions of different temperatures established in the vessel of Fig. 2 according to an embodiment.

Fig. 3C is a schematic diagram illustrating a local accumulation of nucleic acid sequence(s) established in the vessel of Fig. 2 according to an embodiment.

Fig. 4 is a schematic diagram illustrating temperature cycling by circulating fluid flows in a method according to an embodiment.

Fig. 5 is a schematic diagram illustrating the mechanisms that give rise to a spatially varying concentration in a method according to an embodiment.

Fig. 6 is a graph showing the concentration enhancement attainable in a method according to an embodiment.

Fig. 7 is a schematic view of an apparatus according to another embodiment.

Fig. 8 is a greyscale representation of a velocity field established in a capillary using the apparatus of Fig. 7.

Fig. 9 is a view illustrating flow paths established in a vessel with the apparatus of Fig. 7 taking into account thermophoretic drift.

Fig. 10A are views illustrating a local concentration enhancement of DNA obtained in a vessel with the apparatus of Fig. 7 for different times, and Fig. 10B is a graph representing a maximum concentration of the DNA as a function of time.

Fig. 11 are views illustrating a local concentration enhancement of replicated DNA obtained in a vessel with the apparatus of Fig. 7 for different times.

Fig. 12 is a schematic view of a vessel for illustrating a spatio-temporal pattern in which heating power is supplied in an embodiment.

Fig. 13 is a schematic diagram illustrating temperature variations and fluid flows in a vessel when heating power is supplied to the vessel according to the method illustrated in Fig. 12.

Fig. 14A and 14B are graphs representing a temperature variation in a transverse direction of the vessel of Fig. 13 at the positions indicated at 121 and 122 in Fig. 13, respectively.

Fig. 15 is a schematic diagram illustrating processes when heating power is supplied to the vessel according to the method illustrated in Fig. 12.

In that embodiment of the invention there is a discrimination between ssDNA molecules binding to the targets and molecules non-binding to the target (145/146 vs. 142, 143, 144). 131 denotes an ssDNA pool comprising in positions 132 and 133 stretches complementary to the 5' and 3' primer. 134 denotes a carrier on which various targets are immobilized.

While methods and apparatuses according to various embodiments of the invention will be described with reference to the drawing below, it is to be understood that the embodiments are intended to be illustrative for the invention as defined by the claims and are not to be construed as limiting the claim scope.

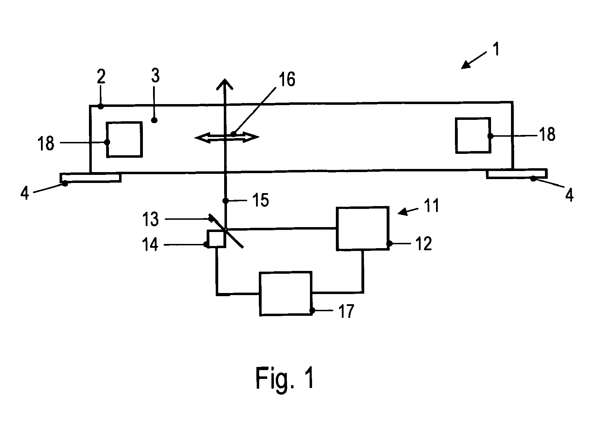

Fig. 1 is a schematic view of an apparatus according to an embodiment. The apparatus 1 comprises support members 4 on which an elongated vessel 2 may be supported. The elongated vessel 2 may be a capillary. The vessel 2 may be open at its longitudinal ends, or may be closed at its longitudinal ends. The vessel 2 may be made of fused silica, borosilicate or another material which exhibits little absorption in a wavelength range which includes the wavelength of the electromagnetic radiation that is used to supply heating power to a solution contained in the vessel 2. The vessel 2 may also be made of another material, for example a plastic material, which exhibits little absorption in a wavelength range which includes the wavelength of the electromagnetic radiation that is used to supply heating power, and that is inert to reactions with the components of the PCR buffer solution.

The vessel 2 contains a PCR buffer solution comprising one or more target nucleic acid sequence(s), a nucleic acid polymerase, NTPs, and primers. The PCR buffer solution may be any one of a number of conventional PCR buffer solutions, such as a FastPCR master mix (Qiagen).

The apparatus 1 further includes a power supply means 11 which comprises a source of electromagnetic radiation 12. A scanning device allows an output beam 15 of the source of electromagnetic radiation 12 to be directed into the vessel 2 and to be moved at least along a longitudinal direction of the vessel 2, as indicated by arrow 16. The scanning device may include, for example, a mirror 13 on which the beam 15 is received, and an actuator 14 coupled to the mirror 13 for adjusting the mirror 13. Other arrangements that allow the beam 15 to be displaced relative to the vessel 2 may also be used. The power source of electromagnetic radiation 12 is configured such that it outputs electromagnetic radiation having a wavelength that is absorbed by the PCR buffer solution, so that the PCR buffer solution is locally heated when the beam 15 passes therethrough. The beam 15 may be focussed such that, upon passage through the vessel 2, it has a diameter that is smaller than the inner width of the vessel 2 in the transverse direction of the vessel which is perpendicular to the propagation direction of the beam. In an embodiment, the source of electromagnetic radiation 12 may be an IR laser. In an embodiment, the IR laser may output electromagnetic radiation having a wavelength of at least 1000 nm. In an embodiment, the IR laser may output electromagnetic radiation having a wavelength of at least 1900 nm, in particular of about 1940 nm.

The apparatus 1 further includes a control device 17 which is coupled to the scanning device 13, 14 and the source 12 of electromagnetic radiation to control an output power of the source 12 of electromagnetic radiation and, via the scanning device 13, 14, the position at which the beam 15 enters the vessel 2. By displacing the beam 15 relative to the vessel 2, the PCR buffer solution may be heated at various positions in a time-varying manner.

The apparatus 1 further includes a temperature adjusting means which may comprise heating members 18, as indicated in Fig. 1, and/or cooling members. The heating members 18 may comprise Peltier elements or any other device or member adapted to adjust an offset temperature of the PCR buffer solution 3 in the vessel 2. The temperature adjusting means may further include an immersion solution surrounding at least part of the vessel 2, to more uniformly heat or cool the vessel 2.

The apparatus 1 is configured such that the power supplied to the PCR buffer solution within the vessel 2 is sufficiently large to establish a temperature variation within the vessel, such that a temperature difference between a peak temperature, which is normally established in a proximity of the positions at which the PCR buffer solution is locally heated, and positions at at least one side wall of the vessel 2 is approximately equal to the difference between the temperature at which a denaturation of the nucleic acid sequence(s) to be replicated and the temperature at which an annealing takes place. A time-averaged output power of the source 12 may be adjusted in dependence on the specific nucleic acid sequence(s) which is or are to be replicated. For illustration, for replicating a 143mer DNA, the apparatus 1 may be configured such that at least a temperature difference of approximately 27°C or more may be established. The temperature adjusting means 18 are configured to adjust a temperature offset in the PCR buffer solution 3 by heating or cooling the PCR buffer solution 3 such that, in at least one region within the vessel 2, the PCR buffer solution has a temperature at which a denaturation of the nucleic acid sequence(s) which is or are to be replicated takes place, and in at least one other region within the vessel 2, the PCR buffer solution has a temperature at which an annealing and elongation step of a PCR take place.

The apparatus 1 is further configured such that the beam 15 of electromagnetic radiation is displaced relative to the vessel 2 as a function of time. The beam may be displaced along the longitudinal direction of the vessel 2. Thereby, energy is supplied to the PCR buffer solution 3 at a plurality of positions within the vessel 2. As can be seen from Fig. 1, energy will generally be supplied to the PCR buffer solution within a cylindrical volume extending through the vessel interior in the propagation direction of the beam 15 and having a diameter corresponding to the diameter of the beam 15 in the vessel 2. The fact that power may be supplied to various positions disposed along the longitudinal direction of the vessel 2 in a time-varying manner does therefore not preclude that heating energy is supplied to the PCR buffer solution at plural positions at any given time.

As has been described by F. M. Weinert and D. Braun in the article "Optically driven fluid flow along arbitrary microscale patterns using thermoviscous expansion", J. Appl. Phys. 104, 104701 (2008), by locally supplying heat to a fluid in a spatio-temporally varying manner, a fluid flow may be established in the fluid. This effect has been attributed to the interplay of thermal expansion and viscosity changes in the front and wake of a laser spot.

A flow velocity of the fluid flow may be approximated by

$$ u_{flow} \approx -k \cdot f \cdot \alpha \cdot \beta \cdot b \cdot \left( \Delta T_0 \right)^2, \qquad (5) $$

where $u_{flow}$ denotes the fluid flow velocity at the position at which heat is supplied to the fluid, f denotes the frequency at which heat is supplied to the fluid at the respective position,

$$ \alpha = \frac{1}{\rho} \cdot \frac{\partial \rho}{\partial T} \qquad (6) $$

denotes the thermal expansion coefficient with $\rho$ being the fluid density and T the temperature,

$$ \beta = \frac{1}{\eta} \cdot \frac{\partial \eta}{\partial T} \qquad (7) $$

denotes the temperature coefficient in the temperature-dependent change in viscosity η of the fluid, b denotes the characteristic radius of the spot at which energy is supplied to heat the fluid, and $\Delta T_0$ denotes a temperature change effected by supplying heating energy at the respective position. In Equation (5), k is a factor that depends on the spatial temperature variation in a circumference of the position at which heating energy is respectively supplied. For a Gaussian temperature variation, $k = 3\sqrt{\pi}/4$. In Equation (5), the temperature change $\Delta T_0$ is determined by the amount of heating energy supplied at a position through which the beam of electromagnetic radiation is scanned upon one passage of the beam through this position. This amount of energy may be controlled by controlling the output power of the source 12 of electromagnetic radiation. For positive coefficients α and β in Equation (5), the fluid flow will be directed opposite to the direction of motion of the beam which supplies heating energy, as indicated by the minus-sign on the right-hand side of Equation (5).

The apparatus 1 is configured such that in a region in which the beam 15 passes through the PCR buffer solution and supplies heating energy to the PCR buffer solution, the PCR buffer solution has a temperature well above 4 °C, typically on the order of 80-95°C. For conventional PCR buffer solutions, the thermal expansion coefficient α and the coefficient β in Equation (5) are non-zero at these temperatures. Thus, a finite fluid flow may be established by heating the PCR buffer solution in a time-varying, in particular a time-sequential, manner at a plurality of positions.

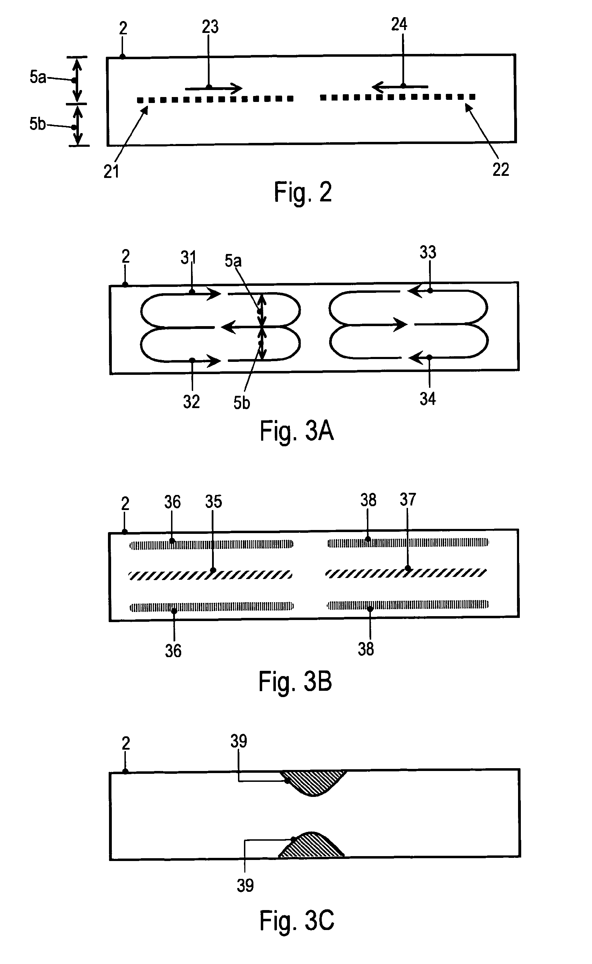

With reference to Figs. 2-5 below, processes that take place in the vessel 2 when heating power is locally supplied to the PCR buffer solution 3 will be described in more detail. In particular, as will be explained, a circulating fluid flow may be established in the PCR buffer solution 3 within the vessel 2, so that at least a fraction of the PCR buffer solution cyclically travels between the regions of different temperatures within the vessel 2.

Driving a fluid flow by time-varying heating

Fig. 2 is a schematic view of the vessel 2 of Fig. 1 as viewed in the propagation direction of the beam 15 (i.e., from top in Fig. 1). A characteristic width of convectional fluid flows in the direction tangential to the fluid flow at the fluid inversion points, which is denoted by "a" herein, is indicated at 5a and 5b. It is noted that the transverse widths of plural convectional fluid flows need not necessarily be identical. If local heating power is supplied along positions located on a centre-plane of the vessel, the width of the convectional fluid flows may be approximated by half the vessel width in the direction perpendicular to the centre-plane.

One spatio-temporal pattern along which the beam 15 may be moved in an exemplary embodiment is illustrated in Fig. 2. According to the exemplary embodiment, the beam 15 is scanned essentially along the longitudinal direction of the vessel 2. The scanning of the beam 15 is performed such that power is supplied at a first plurality of positions 21 in a time-sequential manner, the beam 15 being displaced from one of the first positions 21 to another one of the first positions 21 in a first direction 23. The scanning of the beam 15 is further performed such that power is supplied at a plurality of second positions 22 in a time-sequential manner, the beam 15 being displaced from one of the second positions 22 to another one of the second positions 22 in a second direction 24 which is essentially opposite to the first direction 23. A scanning pattern as indicated in Fig. 2 may be implemented, for example, by controlling the actuator 14 such that the mirror 13 is tilted in one direction in a step-wise fashion until the beam 15 passes through the rightmost position of the first positions 21 in Fig. 2; thereafter, controlling the source 12 such that the output power is essentially zero while controlling the actuator 14 such that the mirror 13 continues to be tilted in the one direction; thereafter, controlling the source 12 such that it again has a finite energy output and controlling the actuator 14 such that the mirror 13 is tilted in another direction, opposite to the one direction, in a step-wise fashion until the beam 15 passes through the leftmost position of the second positions 22 in Fig. 2; thereafter, controlling the source 12 such that the output power is essentially zero while controlling the actuator 14 such that the mirror 13 continues to be tilted in the other direction. Thereafter, the scanning cycle may be repeated.

It should be noted that the scanning pattern shown in Fig. 2 is provided for illustration rather than limitation. Other scanning patterns may be implemented in other embodiments. For further illustration, while the plurality of first positions 21 and the plurality of second positions 22 are shown to be pair-wise spaced from each other in Fig. 2, the first positions 21 and the second positions 22 may also be arranged so as to respectively form continuous line segments.

Fig. 3A is a schematic diagram illustrating a fluid flow established in the vessel 2 when the beam 15 is scanned along the vessel 2 in, for example, the scanning pattern illustrated in Fig. 2. As has been explained with reference to Equation (5) above, scanning the beam 15 through the first positions 21 has the effect of locally driving the PCR buffer solution, the velocity at the first positions 21 being generally directed opposite to the first scan direction 23. The flow velocity has a magnitude which is controllable by controlling the rate at which the beam 15 passes through each one of the first positions 21 and the temperature increase that is respectively attained at the first positions 21 upon passage of the beam 15. As the PCR buffer solution is driven only over a finite length along the longitudinal direction of the vessel 2, return flows along the vessel side walls emerge, giving rise to circulating flows 31 and 32 within the vessel. Similarly, scanning the beam 15 through the second positions 22 has the effect of locally driving the PCR buffer solution, the velocity at the second positions 22 being generally directed opposite to the second scan direction 24 and having a magnitude which is controllable by controlling the rate at which the beam 15 passes through each one of the second positions 22 and the temperature increase that is respectively attained at the second positions 22 upon passage of the beam 15. As the PCR buffer solution is driven only over a finite length along the longitudinal direction of the vessel 2, circulating flows 33 and 34 emerge. The rotation direction of the circulating flow 31 is opposite to the rotation direction of the circulating flow 33 adjacent to the circulating flow 31 in the longitudinal direction of the vessel 2. Similarly, the rotation direction of the circulating flow 32 is opposite to the rotation direction of the circulating flow 34 adjacent to the circulating flow 32 in the longitudinal direction of the vessel 2.

Temperature cycling

Fig. 3B is a schematic diagram illustrating regions of different temperatures established in the vessel 2 when the beam 15 is scanned along the vessel 2 in, for example, the scanning pattern illustrated in Fig. 2.

By scanning the beam 15 through the plurality of first positions 21, the PCR buffer solution in a first region 35 located in proximity to the first positions 21 is heated to a temperature that is higher than a temperature in second regions 36 that are offset relative to the first region 35 in the transverse direction of the vessel 2. The first region 35 and the second regions 36 will typically extend through the vessel in the direction normal to the image plane of Fig. 2. Similarly, by scanning the beam 15 through the plurality of second positions 22, the PCR buffer solution in a first region 37 located in proximity to the second positions 22 is heated to a temperature that is higher than a temperature in second regions 38 that are offset relative to the first region 37 in the transverse direction of the vessel 2. The first region 37 and the second regions 38 will typically extend through the vessel in the direction normal to the image plane of Fig. 2. As has already been explained above, the temperature adjusting means and the source 12 supplying heating power to the PCR buffer solution are configured such that in the first regions 35 and 37 the PCR buffer solution has a temperature at which a denaturation of the nucleic acid sequence(s) which is or are to be replicated takes place, and in the second regions 36 and 38 the PCR buffer solution has a temperature at which an annealing and elongation step of a PCR take place.

Fig. 3A in combination with Fig. 3B illustrate that, by supplying energy to the PCR buffer solution 3 in the vessel 2 at a plurality of positions 21 and 22, respectively, circulating fluid flows 31-34 are established. PCR buffer solution circulating in the flow paths 31-34 experiences a temperature cycling, as the flow paths 31-34 traverse both a first region 35 or 37, respectively, having an elevated temperature and a second region 36 or 38, respectively, having a lower temperature. Thereby, the PCR temperature cycling is implemented.

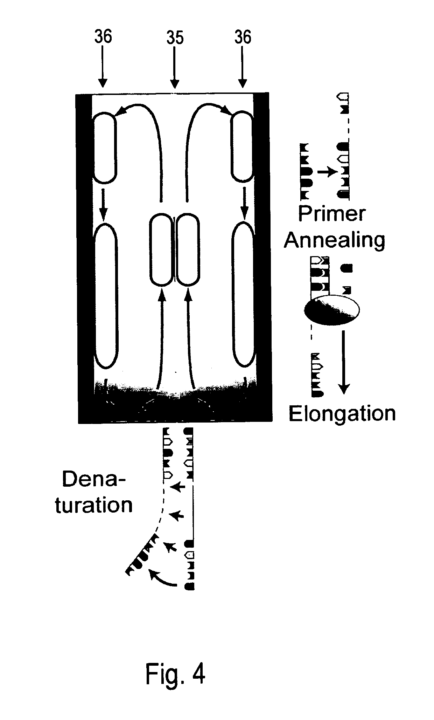

Fig. 4 is a schematic diagram illustrating the temperature cycling in the vessel 2. Only one half of the vessel 2 is illustrated in Fig. 4. As schematically illustrated in Fig. 4, in the first region 35 within the vessel the PCR buffer solution has a temperature at which a denaturation step of a PCR takes place. In the second regions 36, the PCR buffer solution has a temperature at which annealing and elongation steps of a PCR take place.

Concentration variation (local accumulation)

With continued reference to Figs. 2, 3A and 3B, for the PCR buffer solution having a spatially varying temperature, a thermophoretic drift will be established between the regions in which the PCR buffer solution has different temperature. The magnitude of the thermophoretic drift of a molecule or particle in the PCR buffer solution is characterized by the thermodiffusion coefficient $D_T$ of the respective molecule or particle comprised by the PCR buffer solution. The current density due to thermophoretic drift is

$$j_T = -D_T \cdot \nabla T \cdot c, \qquad\qquad (8)$$

where $\nabla T$ is the temperature gradient and $c$ the concentration of the respective molecule or particle the PCR buffer solution. For the temperature variation established by locally supplying energy to a plurality of positions disposed along the longitudinal direction of the vessel 2, the thermophoretic drift will be directed essentially in the transverse direction of the vessel 2. Referring to Fig. 3B, a thermophoretic drift will be established between the first region 35 and the second regions 36, and a thermophoretic drift will be established between the first region 37 and the second regions 38, respectively.

For DNA molecules in conventional PCR buffer solutions, the thermodiffusion coefficient is frequently found to be positive. The thermophoretic drift is then directed from the first region 35 or 37 having a higher temperature to the second regions 36 or 38, respectively, having a lower temperature.

The interplay of thermodiffusion in the transverse direction of the vessel 2 and the bi-directional flow along flow paths 31-34 gives rise to a local increase in concentration of molecules or particles in accumulation regions 39, which are located in between the circulating flows 31 and 33 and in between the circulating flows 32 and 34. The regions 39 in which the concentration of molecules or particles is locally increased are schematically illustrated in Fig. 3C.

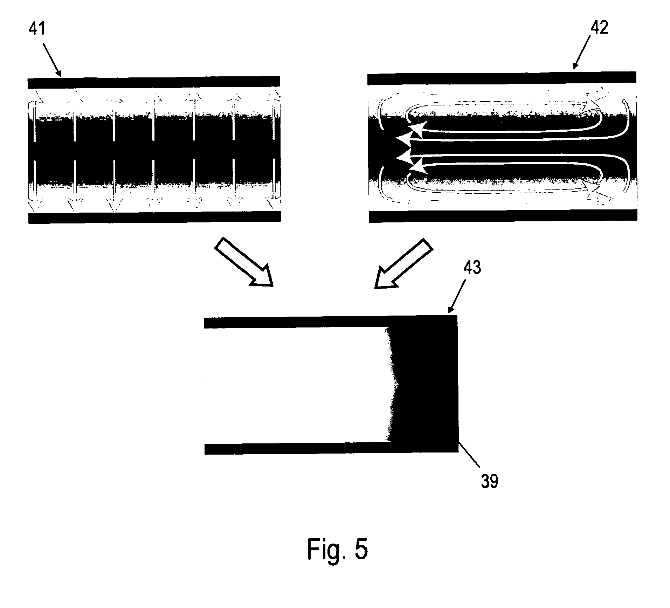

Fig. 5 is a schematic diagram illustrating the interplay of thermodiffusion and circulating fluid flows established by locally supplying energy at plural positions disposed along the longitudinal direction of the vessel 2. Only one half of the vessel 2 is illustrated in Fig. 5.

In graph 41 of Fig. 5, a thermophoretic drift is indicated by arrows. The greyscale background illustrates the temperature variation within the vessel, with darker portions indicating higher temperatures. For molecules or particles having a positive thermodiffusion coefficient, the thermophoretic drift is directed from the plane along which the beam 15 is scanned toward the lateral walls of the vessel, in a direction transverse to the plane along which the beam 15 is scanned.

In graph 42 of Fig. 5, the circulating fluid flow is indicated by arrows. The greyscale background illustrates the temperature variation within the vessel, with darker portions indicating higher temperatures.

In graph 43 of Fig. 5, a concentration of a molecule that exhibits thermophoretic drift is indicated in a greyscale representation. In graph 43, darker colours indicate a higher concentration, and lighter colours represent a lower concentration. The combination of the thermophoretic drift indicated in graph 41 and the circulating fluid flows indicated in graph 42 lead to a local increase in concentration of the respective molecule or particle in a region 39 in the vessel 2, which is disposed at a central position of the vessel, i.e., spaced from the ends of the vessel 2.

While the interplay of thermophoretic drift and a circulating fluid flow gives rise to a local increase in concentration in an accumulation region 39 within the vessel, both the fluid flow circulating within the vessel and diffusion will allow molecules or particles accumulated in the accumulation region 39 to leave the accumulation region 39 at least for some time before being transported back into the accumulation region 39. Therefore, the PCR may be performed even when the concentration of the replicated nucleic acid sequence(s) exhibits pronounced variations in concentration.

Control of accumulation

The mechanism described with reference to Figs. 3B, 3C and 5, which transports molecules or particles toward specific accumulation regions 39, is counterbalanced by diffusion. The local increase in concentration is controllable by adjusting the flow velocity of the fluid flows 31-34 circulating within the vessel 2.

The functional dependence of the increase in concentration in the accumulation regions 39 on the fluid flow velocity may be estimated by

$$\frac{c_{acc}}{c_0} \approx \exp\left(\frac{504 \cdot v_c \cdot a \cdot S_T \cdot \Delta T \cdot r \cdot D}{2835 \cdot D^2 + 128 \cdot v_c^2 \cdot a^2}\right), \tag{9}$$

where $c_{acc}$ denotes a concentration of a molecule, complex or particle in the accumulation regions, e.g., the maximum concentration of the molecule, complex or particle in the vessel; $c_0$ denotes a concentration of the respective molecule, complex or particle at the longitudinal ends of the elongated vessel; $v_c$ denotes the maximum flow velocity of the circulating fluid flows, which may, for example, be estimated to be 2/3 of the actual maximum velocity in the three-dimensional vessel; "a" again denotes the characteristic transverse width of a convectional fluid flow circulating within the vessel; $S_T = D_T/D$ denotes the Soret coefficient for the respective molecule, complex or particle; $\Delta T$ denotes the temperature difference between the first region 35 or 37 and the second regions 36 or 38, respectively; $r = l/a$, where $l$ is the length of the circulating fluid flow taken along the vessel longitudinal direction, which is determined by the plurality of positions at which local heating power is supplied; and D denotes the diffusion coefficient of the respective molecule, complex or particle in the PCR buffer solution.

As can be seen from the estimate in Equation (9), the local increase in concentration in the accumulation region, i.e., the quotient $c_{acc}/c_0$, is a function of both the maximum flow velocity of the circulating fluid flows and the diffusion coefficient of the respective molecule, complex or particle. It will be appreciated that the local increase in concentration may be controlled by controlling the velocity of the fluid flows circulating within the vessel.

Further, the velocity $v_{c\text{-}max}$ at which the right-hand side of Equation (9) becomes maximum is a function of D/a. For molecules or particles having different diffusion coefficients, the local increase in concentration in the accumulation region may be made more pronounced for one type of molecule, complex or particle than for other types of molecules, complexes or particles by adjusting the velocity $v_c$ of the fluid flows circulating within the vessel.

In the apparatus and method described herein, a velocity of the fluid flow may be adjusted by controlling the spatio-temporal pattern in which heating power is supplied to the PCR buffer solution. For illustration, referring to Equation (5), a maximum flow velocity of the PCR buffer solution within the vessel may be adjusted by controlling the output power of the source 12 of electromagnetic radiation and/or by adjusting the rate f at which power is respectively supplied at the plurality of positions and/or by controlling the spatio-temporal heating pattern. The apparatus and method according to various embodiments allow the local increase in concentration of a molecule, complex or particle to be controlled via the spatio-temporal pattern according to which heating power is supplied to the PCR buffer solution. Further, the accumulation may be made more pronounced for one type of molecule, complex or particle than for other types of molecules, complexes or particles by controlling the spatio-temporal heating pattern.

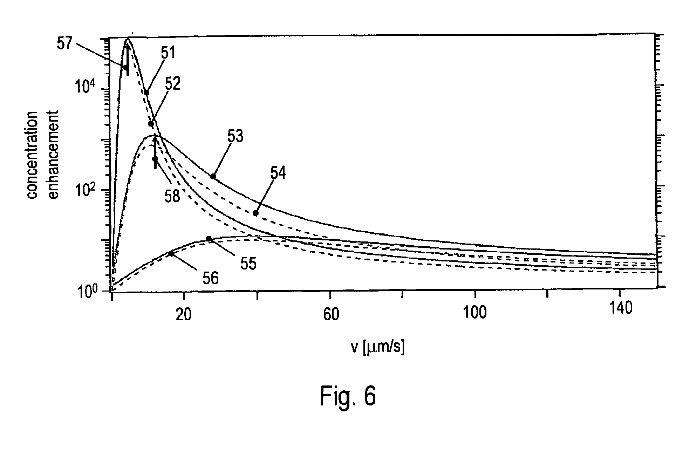

Fig. 6 is a graph illustrating, for three different types of molecules, the normalized increase in concentration $c_{acc}/c_0$ as a function of the maximum velocity $v_c$ of the fluid flow circulating within an elongated vessel having a rectangular cross section. The results of two-dimensional numerical simulations are shown in solid lines, while the right-hand side of Equation (9) is respectively shown in broken lines. The results have been obtained for a fluid flow having a transverse width of 50 $\mu$m. Lines 51 (simulation) and 52 (analytical expression) illustrate the concentration enhancement as calculated for a 100mer dsDNA having a diffusion coefficient of D=45 $\mu$m$^2$/s. A maximum enhancement of the concentration is expected for $v_{c-max} \approx 4{,}92$ $\mu$m/s indicated at 57. Lines 53 (simulation) and 54 (analytical expression) illustrate the concentration enhancement as calculated for a 22mer ssDNA having a diffusion coefficient of D=115 $\mu$m$^2$/s. A maximum enhancement of the concentration is expected for $v_{c-max=}11{,}2$ $\mu$m/s indicated at 58. Lines 55 (simulation) and 56 (analytical expression) illustrate the concentration enhancement as calculated for single nucleotides having a diffusion coefficient of D=400 $\mu$m$^2$/s. A maximum enhancement of the concentration is expected for $v_{c-max} \approx 39{,}5$ $\mu$m/s.

In a vessel containing a PCR buffer solution comprising, for example, 100mer dsDNA, 22mer ssDNA and single nucleotides, by establishing a fluid flow within the vessel having a velocity of $v_{c-max} \approx 4{,}92$ $\mu$m/s, a pronounced local increase in concentration can be attained for the 100mer dsDNA in an accumulation region, while the local increases in concentration are less pronounced for the 22mer ssDNA and single nucleotides.

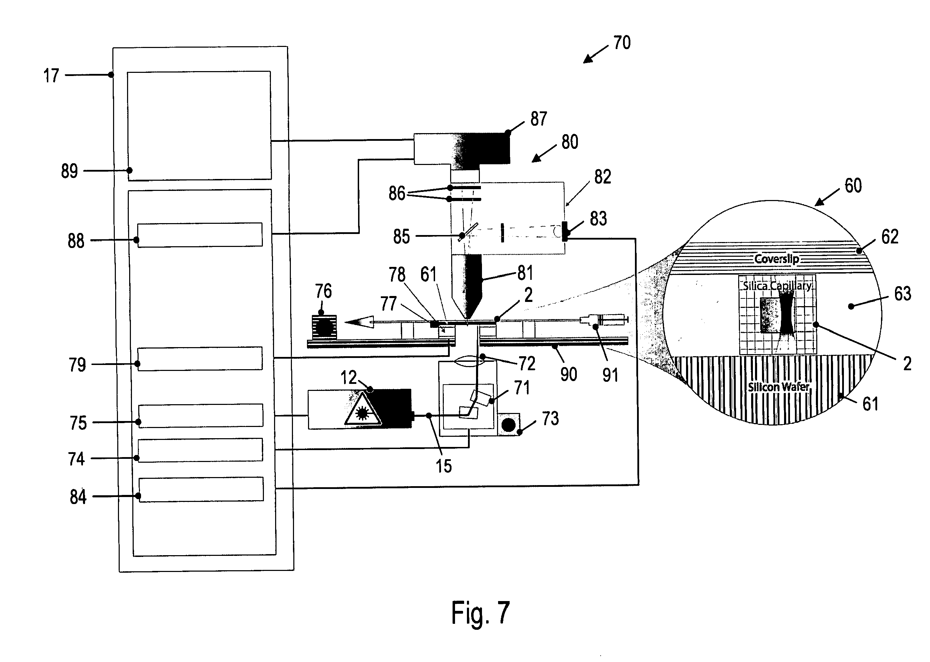

With reference to Figs. 7-15, apparatuses and methods according to further embodiments of the invention will be described in more detail. The operation of these apparatuses and methods is based on the mechanisms described with reference to Figs. 1-6 above, and reference is also made to the explanations given with reference to Figs. 1-6.

Fig. 7 is a schematic view of an apparatus 70 according to another embodiment. The inset 60 shows an enlarged cross-sectional view taken in a plane perpendicular to a longitudinal axis of a vessel.

As best seen in the inset 60, the vessel 2 is configured as a silica capillary having a square inner diameter. An inner width of the vessel 2 may be, for example, 50 $\mu$m. The vessel 2 is supported on a silicon wafer 61, which allows the output beam 15 of a source 12 of electromagnetic radiation to pass therethrough. Immersion oil 63 is provided in the space between the silicon wafer 61 and a coverslip 62. The vessel 2 may be releasably supported on the silicon wafer 61, so that the vessel 2 can be exchanged when required.

The apparatus 70 further comprises the source 12 of electromagnetic radiation, which in this case is configured as an IR laser having an output wavelength of 1940 nm, a pair of x-y-mirrors 71 that allow the output beam 15 of the source 12 of electromagnetic radiation to be deflected, a laser lens 72 for focussing the beam 15 in the capillary and a heat bath 73 for suppressing thermal expansion of the x-y-mirrors 71 and/or laser lens 72. A laser control 75 allows the laser output power to be controlled, e.g., by switching the laser output on and off. An x-y-mirror control 74 controls the deflection of the beam 15 and, thereby, the position at which the beam 15 passes through the vessel 2. As has been explained with reference to Figs. 1-6, the source 12 of electromagnetic radiation in combination with the scanning arrangement implemented by the x-y-mirrors 71 make it possible to locally supply heating power to a solution within the vessel at a plurality of positions in a time-varying manner. The time-averaged output power of the IR-laser is adjusted so as to attain a desired temperature difference between different regions within the vessel 2.

The apparatus 70 further includes a temperature adjusting means which allows an offset temperature of the solution within the vessel 2 to be adjusted. The temperature adjusting means comprises Peltier elements 78 which are controlled by a Peltier control 79. The silicon wafer 61 is positioned in thermal contact with the Peltier elements 78, and heat is also transferred from the silicon wafer 61 to the immersion oil 63 which comes into contact with the longitudinal side walls of the vessel 2. Via the Peltier elements 78, an offset temperature may be adjusted in the solution within the vessel. Heat may be transferred to the vessel 2 via the Silicon wafer 61 or the immersion oil 63. The temperature adjusting means in combination with the local heating provided by scanning the beam 15 through the vessel 2 allows a spatially varying temperature to be established within the vessel such that, in a first region within the vessel, the solution has a temperature at which a denaturation step of a PCR takes place, and in a second region within the vessel, the solution has a temperature at which an annealing and elongation step, respectively, of a PCR take place.

A heat bath 76 reduces thermal expansion of the microscopy stage due to, e.g., laser absorption. The heat bath may also allow the peltier elements to hold the probe temperature at a constant level over an extended period of time.

The apparatus 70 further comprises optical components that allow progress of a PCR to be directly monitored, for example, in a real-time PCR. A fluorescence microscope 82 comprises a LED 83, e.g., a cyan LED, for illumination. The LED 83 is controlled by a LED control 84. Optical components, such as air optics 81 for focussing a signal from the solution within the vessel, a semitransparent mirror 85 and suitable filters 86, like an emission-/absorption and IR filter, allow the solution within the vessel 2 to be imaged. A CCD camera 87 provides captured image data to an image capture tool 89. The CCD camera 87 is triggered by a CCD-camera trigger 88.

One or several of the x-y-mirror control 74, the laser control 75, the Peltier control 79, the LED control 84, the CCD-camera trigger 88 and the image capture tool 89 may be implemented by software, hardware or a combination of both in a computer 17. The computer 17 may be embedded in the apparatus 70.

The apparatus 70 further comprises a pump 91. The pump 91 allows a net flow of PCR solution to be established through the vessel 2.

In the apparatus 70, one or usually at least two fluid flows circulating within the vessel 2 may be established in the manner described with reference to Figs. 1-6, i.e., by scanning the output beam 15 of the IR laser through a plurality of positions. The level of control provided when the fluid flows are driven by locally heating the solution within the vessel 2 in a time-varying manner allows the various processes described with reference to Figs. 1-6, including a temperature cycling realized by the fluid flows circulating within the vessel and a local accumulation of specific molecules, complexes or particles, to be realized within the vessel 2. In the apparatus 70, such processes may be monitored using the fluorescence microscope 82.

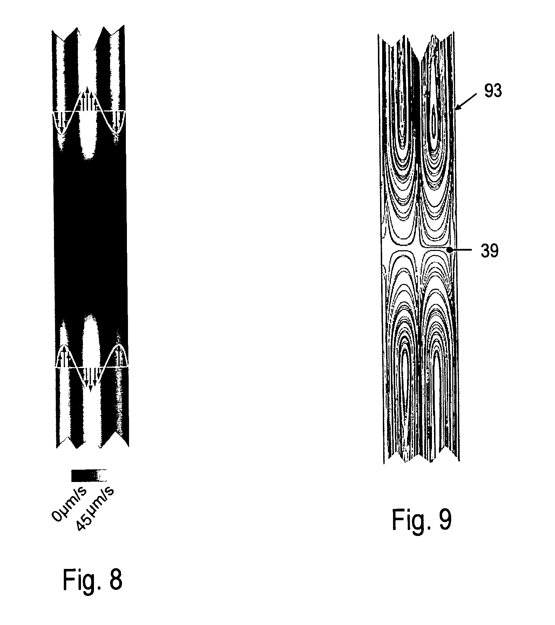

Fig. 8 is a greyscale representation of a velocity field established in the vessel 2 using the IR laser and scanning components of the apparatus of Fig. 7. A borosilicate capillary having a square cross section of 100 $\mu$m $\times$ 50 $\mu$m and a length of 1800 $\mu$m has been sealed on both ends with a reservoir of paraffin oil. The solution in the capillary has been a commercial FastPCR master mix (Qiagen). The laser beam has been scanned along the capillary longitudinal axis as described with reference to Fig. 2. To estimate the fluid flow velocity, 1 $\mu$m fluorescent beads (F8888, Invitrogen) were imaged in the initial 5 seconds after the laser had been switched on. The velocity field is shown in Fig. 8 in a greyscale representation. The flow direction is respectively indicated by arrows. A maximum flow velocity of approximately 70 $\mu$m/s has been recorded.

Fig. 9 shows molecular pathways in the silica capillary which result from the velocity field of Fig. 8 and thermophoretic drift for 143mer DNA. The thermophoretic drift has been determined based on temperature imaging performed with the dye BCECF ((2',7'-bis-(2-carboxyehtyl)-5-(and-6)-carboxyfluorescein, Fermentas). As is seen in Fig. 9, there are molecular pathways which terminate at the lateral walls of the capillary in a centre region of the capillary. This leads to a local increase in concentration of the 143mer DNA in an accumulation region indicated at 39.

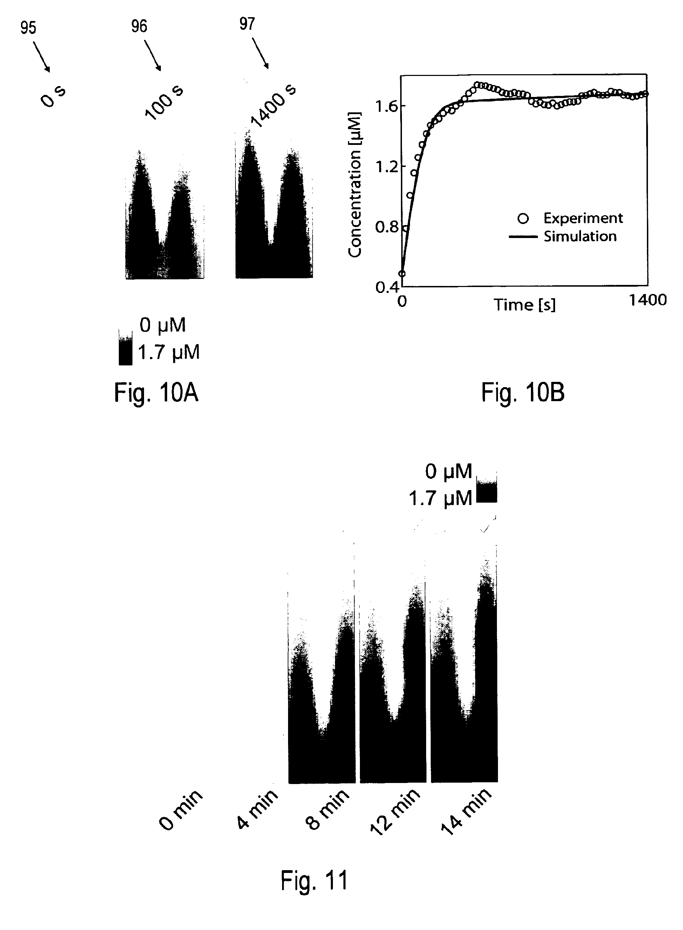

Fig. 10A shows a graph illustrating the concentration of a 143mer DNA as seen in fluorescence images at three different times, using the same setting as for Figs. 8 and 9. Only one half of the capillary is shown in Fig. 10A, the lower end of the fluorescence images corresponding to a central portion of the capillary. Fluorescence image data 95 show the concentration of the 143mer DNA at time t=0. The concentration is essentially uniform. Fluorescence image data 96 show the concentration of the 143mer DNA at time t=100 s. At t=100 s, there is already a noticeable increase in local concentration at a central portion of the capillary. Fluorescence image data 97 show the concentration of the 143mer DNA at time t=1400 s. Pronounced local increases in concentration have developed in an accumulation region located at a central portion of the capillary.

Fig. 10B shows a graph illustrating the measured centre concentration of the 143mer DNA and results of finite element simulations. A significant increase in concentration of the 143mer DNA has been observed over a time of a few hundred seconds.

Fig. 11 shows a graph illustrating the fluorescence signal obtained when heating power is supplied so as to establish conditions in which both a local increase in concentration and a PCR are attained in the reaction vessel. Only data obtained from one half of the capillary is shown in Fig. 11, the lower end of the fluorescence images corresponding to a central portion of the capillary.

The reaction vessel is identical to the one in which the measurements explained with reference to Figs. 8-10 have been performed, i.e., a silica capillary having an inner diameter of 100 $\mu$m $\times$ 50 $\mu$m and a length of 1800 $\mu$m. The vessel contains a solution which consists of 10 $\mu$l FastPCR master mix (Qiagen), 1 $\mu$l of a 1 nM diluted 143mer template, 2 $\times$ 1 $\mu$l primer with a final concentration of 500nM , 1 $\mu$l 10 x SYBR Green I (Molecular Probes, Eugene), 1 $\mu$l 60 mg/ml BSA (biotin free, Roth) to prevent polymerase denaturation at the capillary surface and 6 $\mu$l ultra pure water. An IR laser beam has been scanned along two line segments in a scanning pattern as shown in Fig. 2. The scan parameters have been set so as to establish the velocity field shown in Fig. 8 with a maximum flow velocity of approximately 70 $\mu$m/s. The laser power and a temperature offset have been set so that the solution has a temperature of 86°C in a first region within the capillary (where laser heating takes place) and a temperature of 59°C in second regions offset relative to the first region in the transverse direction of the capillary.

The fluorescence data has been acquired after times of 1 min, 4 min, 8 min, 12 min and 14 min. The fluorescence signal exhibits a pronounced increase over time. The signal varies strongly as a function of location, indicating an accumulation in a centre portion of the capillary.

Assuming a separation of time scales between fast accumulation and slow replication, a concentration $c_{center}$ of the 143mer DNA in the capillary centre is expected to vary with time as

$$c_{center}(t) = \int_0^t c_T(\tau)\dot{c}_{PCR}(t-\tau)d\tau,\qquad\qquad (10)$$

with

$$c_{PCR}(t) = \frac{c_{max}}{1 + \exp[\ln(2)\cdot(t_{1/2} - t)/\tau_d]}$$

where $c_T(t)$ describes the accumulation as a function of time in the absence of a PCR, as shown in Fig. 10B, $\tau_d$ is the doubling time and $c_{max}$ is the maximal concentration to be achieved. By fitting Equation (10) to the measured fluorescence data, a doubling time of $\tau_d \approx 74$ s is obtained. This time is comparable to an average cycling time for the solution in the circulating fluid flows established in the capillary, supporting that the temperature cycling is effected by the circulating fluid flows.

The delays of replication (as obtained by the delta-delta method) allow one to infer an additional duplication time of $\tau_d \approx 50$s by using:

$$t_{doubling} = \frac{\ln(2)\cdot\left(t_{1/2}^B - t_{1/2}^A\right)}{\ln(c_A/c_B)},$$

where $t^B$1/2 denotes a time at which half the maximum fluorescence signal is obtained starting from an initial concentration of $c_B$, and $t^A_{1/2}$ denotes a time at which half the maximum fluorescence signal is obtained starting from an initial concentration of $c_A$. The two values are comparable within the given errors.

As a further control, the measurement has been repeated with a solution which does not comprise the 143mer DNA template. No significant fluorescence signal has been found over a time of 14 min.

As yet a further control, in order to verify that the fluorescence signal shown in Fig. 11 can be attributed to the replicated 143mer DNA, characteristics (in particular thermodiffusion) of the molecules accumulated in the capillary centre after the PCR have been measured using the technique described in WO 2009/141390 A1. The signal has been compared to the signal obtained with a 143mer DNA replicated in a conventional thermocycler. Good agreement has been obtained, supporting that the product accumulated in the capillary centre is the replicated 143mer DNA.

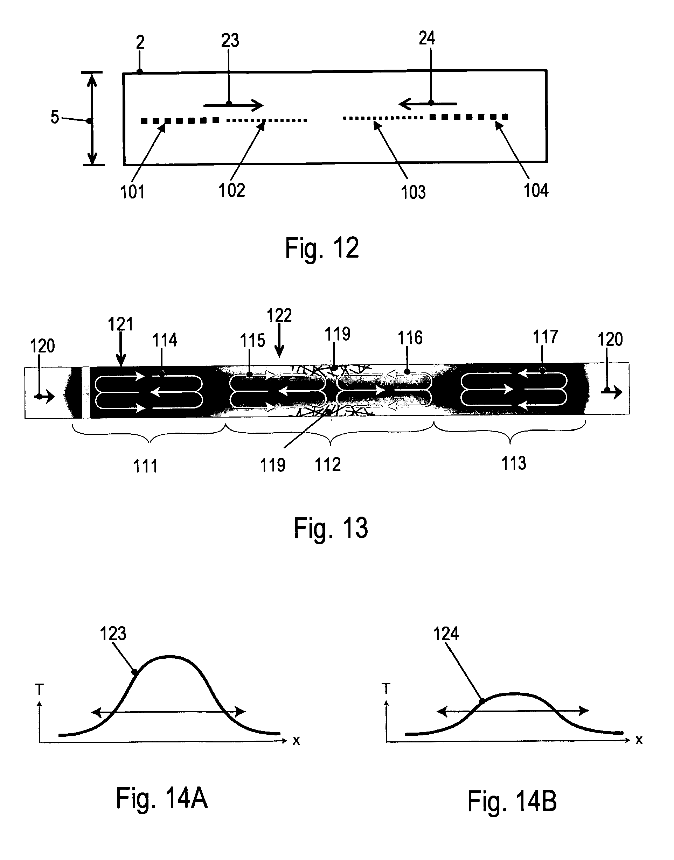

With reference to Figs. 12-15, yet another embodiment of the invention will be described. In the embodiment, an apparatus configured for example as explained with reference to Figs. 1-6 or with reference to Fig. 7 may be utilized for applications such as aptamer synthesis, as already detailed above.

As will be explained in more detail below, in an embodiment, local heating of the solution within the vessel may be performed such that,

- in a first portion of the vessel, the maximum flow velocity of a circulating fluid flow is set based on characteristics, in particular based on a diffusion coefficient, of a molecule, complex or particle, for which a strong accumulation is desired, in particular the target ssDNA complex, and
- in a second portion of the vessel, a temperature variation in the solution is adjusted such that the temperature cycling required for a PCR is realized in the second portion.

Fig. 12 is a schematic view of an elongated vessel 2 having a transverse inner width indicated at 5. The vessel 2 contains a PCR buffer solution comprising one or more nucleic acid sequence(s), a nucleic acid polymerase, NTPs, a first primer and a second primer. In an embodiment, the nucleic acid sequences are ssDNA or ssRNA sequences which comprise random sequences comprising respectively at their 5' and 3' ends fixed sequences for PCR amplification. The PCR solution may be a mutagenic PCR solution and may additionally comprise a plurality of target sequences immobilised on a carrier. In an exemplary embodiment, the PCR solution may further comprise an intercalating dye.

Heating power is supplied to the solution within the vessel 2 at a plurality of positions 101-104 in a time-varying manner, so that the position(s) at which heating power is supplied to the solution varies as a function of time. In the apparatus explained with reference to Figs. 1-6 or in the apparatus explained with reference to Fig. 7, heating power may be supplied in a time-varying manner by temporal scanning of, e.g., an IR laser beam.

As illustrated in Fig. 12, an amount of energy respectively supplied at the various positions may be set so as to vary as a function of position. Thereby, flow velocities of fluid flows circulating within the vessel and temperature gradients established in the transverse direction of the vessel can be controlled separately in different portions along the longitudinal direction of the vessel.

As illustrated in Fig. 12, heating power is supplied to a plurality of positions 101 and 102 such that the position at which the solution is heated moves in a first direction 23. The amount of energy respectively supplied at the positions 101 that are further away from a vessel centre portion is respectively greater than an amount of energy respectively supplied at the positions 102 that are located closer to the vessel centre portion. A heating pattern as illustrated in the left-hand side of Fig. 12 may be realized by scanning a laser beam in the first direction 23 along the vessel longitudinal direction while controlling the laser output power. Similarly, heating power is supplied to a plurality of positions 103 and 104 such that the position at which the solution is heated moves in a second direction 24. The amount of energy respectively supplied at the positions 104 that are further away from a vessel centre portion is greater than an amount of energy respectively supplied at positions 103 that are located closer to the vessel. A heating pattern as illustrated in the right-hand side of Fig. 12 may be realized by scanning a laser beam in the second direction 24 along the vessel longitudinal direction while controlling the laser output power. The laser beam may be scanned over the positions 101 and 102 and over the positions 104 and 103 in a time-sequential manner, the scanning being periodically repeated.

Fig. 13 is a schematic view illustrating fluid flows established in the vessel when a heating pattern as shown in Fig. 12 is implemented, in which the energy supplied to the solution is varied as a function of position. In Fig. 13, temperature is schematically indicated in greyscale, wherein darker colour corresponds to higher temperature. Fluid flows are schematically indicated by arrows within the vessel.

Regions having different temperature variations are established in the vessel 2. A portion 112, in which the locally supplied heating power is smaller than in adjacent portions 111 and 113, exhibits a temperature variation in the transverse direction which is smaller than the temperature variation in the transverse direction in portions 111 and 113 adjacent to the portion 112 in the longitudinal direction of the vessel 2. More specifically, a temperature difference of the solution temperature taken in the lateral centre and the solution temperature taken at the lateral side walls of the vessel is larger for the portions 111 and 113 than for the portion 112.

The different temperature profiles are further illustrated in Figs. 14A and 14B. Fig. 14A schematically illustrates a temperature profile 123 indicating the temperature of the solution as a function of transverse position in the portion 111, as established at the longitudinal position 121 indicated in Fig. 13. Fig. 14B schematically illustrates a temperature profile 123 indicating the temperature of the solution as a function of transverse position in the portion 112, as taken at the longitudinal position 122 indicated in Fig. 13. The temperature profile 123 shows a stronger variation in temperature than the temperature profile 124.

As has been explained above, in particular with reference to Figs. 2 and 3, the local heating in accordance with a spatio-temporal pattern as indicated in Fig. 12 gives rise to fluid flows 114, 115, 116 and 117 circulating within the vessel 2.

As has also been explained above, in particular with reference to Figs. 2, 3, 5 and 6, the counter-rotating fluid flows in the portion 112 in combination with the thermal gradient in the transverse direction of the vessel 2 give rise to a local increase in concentration of molecules, complexes or particles comprised by the solution in an accumulation region 119, which is spaced from the ends of the vessel 2. The amount of local concentration enhancement in the accumulation region 119 depends on the characteristics of the respective molecule, complex or particle, in particular on its diffusion coefficient.

As indicated in Fig. 13, the vessel 2 may be open at its ends, and a net flow 130 of solution may be driven through the vessel 2. The net flow 130 may be driven, for example, by using micropumps.

With continued reference to Figs. 12 and 13, in an embodiment, a power source which locally supplies heating power to the solution and a temperature adjusting means are controlled such that a spatial temperature variation is established in portions 111 and 113 that allows a PCR to take place. The power source is further controlled such that the maximum flow velocities in the circulating fluid flows 115 and 116 are set based on a diffusion coefficient of a molecule, complex or particle, for which a strong accumulation is desired. The maximum flow velocities in the circulating fluid flows 115 and 116 may be set based on the quotient of the diffusion coefficient of a molecule, complex or particle, for which a strong accumulation is desired, and the transverse width "a" of at least one fluid flow circulating within the vessel 2. The maximum flow velocities in the circulating fluid flows 115 and 116 may be set such that $3{,}99 \cdot (D/a) \leq v_c \leq 5{,}39 \cdot (D/a)$, in particular such that $4{,}45 \cdot (D/a) \leq v_c \leq 4{,}92 \cdot (D/a)$, in particular such that $v_c \approx 4{,}69 \cdot (D/a)$, where D denotes the diffusion coefficient of the molecule, complex or particle for which a high local concentration in the accumulation area 119 is desired, and where $v_c$ denotes a vessel-averaged maximum velocity. The vessel-averaged maximum velocity may be approximated to be 2/3 of the actual maximum velocity within the vessel, which may be adjusted via adjusting the manner in which heating power is locally supplied to the solution. The transverse width of the circulating fluid flows may essentially correspond to a distance between the positions 102, 103 and the lateral walls of the vessel 2 offset thereto in a direction perpendicular to the beam of electromagnetic radiation.

With continued reference to Figs. 12-14, a method of synthesising aptamers will be described, in which a temperature distribution and velocity field as exemplarily shown in Figs. 13 and 14 may be utilized.

In the embodiment, the vessel 2 contains a PCR buffer solution comprising nucleic acid sequences, a nucleic acid polymerase, NTPs, a first primer and a second primer. The nucleic acid sequences are ssDNA or ssRNA sequences which comprise random sequences comprising respectively at their 5' and 3' ends fixed sequences for PCR amplification. The PCR solution is a mutagenic PCR solution and additionally comprises a plurality of target sequences immobilised on a carrier. A relation between the first primer and the second primer is between 1:10 and 1:30. This allows an asynchronous PCR to be performed, in which the formation of dsDNA or dsRNA is suppressed. Examples of target molecules are antibodies, labelled nanoparticles etc.

Heating power is supplied to the PCR buffer solution within the vessel, for example using IR laser scanning, such that in portions 111 and 113 a temperature difference of at least 25K, in particular of approximately 35K, is established between the solution at the lateral centre and the solution at the lateral side walls of the vessel. In an embodiment, the heating power supplied at the plurality of positions 101 and 104 and a temperature adjusting means is controlled such that in portions 111 and 113 the solution in a centre region surrounding positions 101 and 104 has a temperature of approximately 90°C, and the solution has a temperature of approximately 55°C in proximity to the lateral walls of the vessel. Temperature cycling between 90°C and 55°C is achieved by the circulating fluid flows 114 and 117.

In the portion 112, heating power is supplied such that a peak temperature of the solution within the portion 112 is smaller than a peak temperature of the solution within the portions 111 and 113, and is adjusted such that, if a complex is formed between the amplified nucleic acid sequence and a specific target sequence immobilised on the carrier, a dissociation rate of the complex is low at the peak temperature within the portion 112. Further, a maximum flow velocity of the circulating fluid flows 115 and 116 within the portion 112 is adjusted, e.g. by controlling the IR laser scanning, such that a pronounced concentration increase of the carrier with the complex between the amplified nucleic acid sequence and the specific target sequence immobilised on the carrier is attained in the accumulation regions 119. This may be attained by controlling the maximum velocity of the fluid flows 115 and 116 such that $3,99 \cdot (D/a) \leq v_c \leq 5,39 \cdot (D/a)$, in particular such that $4,45 \cdot (D/a) \leq v_c \leq 4,92 \cdot (D/a)$, in particular such that $v_c \approx 4,69 \cdot (D/a)$, where D denotes the diffusion coefficient of the complex between the amplified nucleic acid sequence and the specific target sequence immobilised on the carrier, and where $v_c$ is an averaged maximum velocity.

By controlling the maximum velocity of the fluid flows 115 and 116 in the portion 112 as indicated above, a local increase in concentration of the complex between the nucleic acid sequence and the specific target sequence immobilised on the carrier is attained in the accumulation regions 119. ssDNA or ssRNA that has not formed a complex with the specific target exhibits less pronounced variations in concentration and is more likely to diffuse from portion 112 to one of portion 111 and 113, where it will be mutated and replicated. Mutated ssDNA or ssRNA formed in the portion 111 and 113 may move from the portion 111 or 113 to the centre portion 112, for example due to drift or diffusion. In the centre portion 112, the suitably mutated ssDNA or ssRNA may form a complex with the target, depending on its nucleic acid sequence and accumulates in the accumulation region.

A net flow 120 of the solution through the vessel 2 is established, in order to supply new NTPs and nucleic acid polymerase to portions 111 and 113, so that the PCR may be performed over extended periods of time. A velocity of the net flow 120 is set to be smaller than the maximum velocity in the fluid flows 115-117. In exemplary embodiments, the net flow 120 may have a velocity of less than 1 μm/s.

With the above-described process, complexes of ssDNA or ssRNA and the specific target immobilised on the carrier will be accumulated in the accumulation region 119. By use of a mutagenic PCR solution non-binding ss nucleic acid molecules may be modified so as to enable their binding to the specific target molecule. Said ss nucleic acid may be created, for example by a combinatorial library in relation to a not identified biological molecule.

The ssDNA or ssRNA that forms the complex with the specific target may be extracted from the accumulation region 119 and may be subject to further processing. The further processing may include separating the ssDNA or ssRNA from the target, cloning and DNA or RNA sequencing.

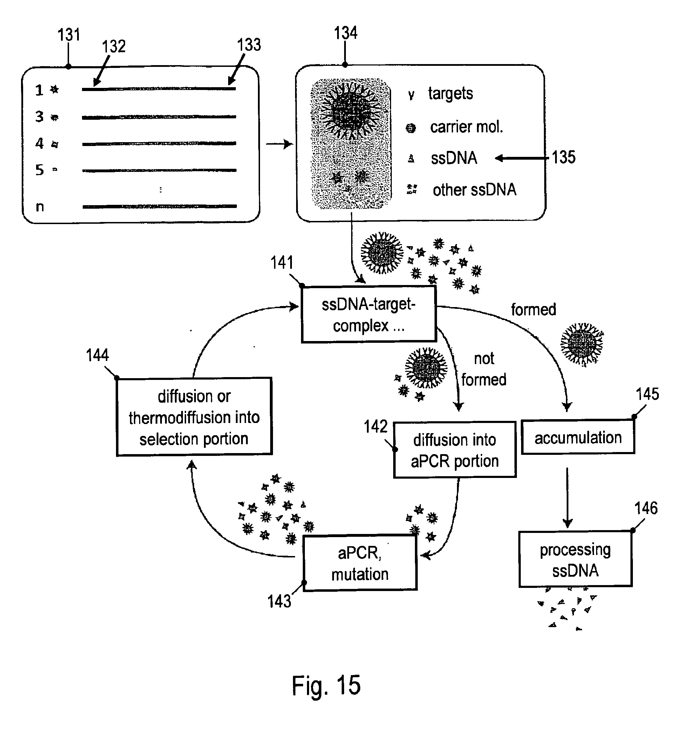

Fig. 15 is a schematic illustration of the above-described process. While the process will be explained for ssDNA with reference to Fig. 15 for illustration, it is to be understood that the process may also be implemented with ssRNA.

A pool of ssDNA or ssRNA is indicated at 131. The pool comprises ssDNA sequences which comprise random sequences comprising respectively at their 5' and 3' ends 132 or 133 fixed sequences for PCR amplification. The solution 134 includes targets, carrier molecules on which the targets are immobilised, and the pool of ssDNA. A ssDNA 135 that can form a complex with the targets is schematically illustrated to have a triangular shape.

Depending on the sequence of nucleic acids, ssDNA may or may form a complex with the target, as indicated at 141.

If a ssDNA-target-complex is formed, the complex between the ssDNA and the target immobilised on the carrier molecule will be accumulated, as indicated at 145. When a sufficient number of complexes between the ssDNA and the target immobilised on the carrier has been accumulated, the complexes may be extracted from the vessel, for example by using electrophoresis, and may be subject to further processing, as indicated at 146. The further processing may include separating the ssDNA or ssRNA from the carrier, cloning and DNA or RNA sequencing.

If a ssDNA-target-complex is not formed, the ssDNA may move to portions of the vessel in which an aPCR is performed, as indicated at 142. For illustration, the ssDNA may diffuse into a portion of the vessel having higher peak temperatures, in which an aPCR is performed.

In the aPCR portion, the ssDNA may be replicated, as indicated at 143. By mutation upon replication, a ssDNA molecule may be obtained that is able to bind to the target on the carrier thus forming a complex.

The replicated and/or mutated ssDNA may move back from the aPCR portion to the selection portion of the vessel, as indicated at 144 depending on its ability to form a complex with the target.

By using the method of the invention for the production of aptamers, binding molecules to virtually any target may conveniently be created in large amounts to be used for therapeutic or diagnostic purposes. Moreover, that reaction may also be used for diagnostic purposes. On the basis of the binding of a selected ssDNA to a specific target, information about a disease state, for example characterized by the up-regulation of receptors, may be obtained.

**Claims**

1. A method of amplifying one or more target nucleic acid sequence(s) in a PCR buffer solution comprising one or more target nucleic acid sequence(s), a nucleic acid polymerase, NTPs, at least a first primer and a second primer, wherein said PCR buffer solution is contained in an elongated vessel (2), said method comprising:

   - establishing a spatially varying temperature (123) in said solution, such that

      o in a first region (35, 37) within said vessel (2), said PCR buffer solution has a temperature at which a denaturation step of a PCR takes place,
      o in a second region (36, 38) within said vessel (2), said PCR buffer solution has a temperature at which an annealing step and an elongation step of a PCR takes place, and
      o said first region (35, 37) and said second region (36, 38) are offset relative to each other in a transverse direction of said vessel (2), and

   - locally supplying heating power to said PCR buffer solution at a plurality of positions (21, 22; 101-104) disposed along a longitudinal direction of said vessel (2) in a time-varying manner to drive said PCR buffer solution so as to establish a fluid flow (31-34; 114, 117) circulating within said vessel (2), such that at least a fraction of said PCR buffer solution cyclically travels between said first region (35, 37) and said second region (36, 38).

2. The method of claim 1, wherein at least one of heating power respectively supplied at said plurality of positions (21, 22; 101-104) and a frequency at which heating power is respectively supplied at said plurality of positions (21, 22; 101-104) is controlled to set a velocity of said PCR buffer solution at said plurality of positions (21, 22; 101-104).

3. The method of claim 1 or claim 2, wherein said locally supplying heating power comprises scanning a beam (15) of electromagnetic radiation, in particular an IR laser beam, in a first direction (23) along a first line segment which extends along a longitudinal direction of said vessel (2), and scanning said beam (15) of electromagnetic radiation in a second direction (24) along a second line segment which extends along said longitudinal direction of said vessel (2), wherein said second direction (24) is opposite to said first direction (23).

4. The method of any one of the preceding claims, wherein said vessel (2) has a rectangular cross section and said heating power is supplied such that said plurality of positions (21, 22; 101-104) are spaced from at least two lateral walls of said vessel (2).

5. The method of any one of the preceding claims, wherein said heating power is supplied such that a velocity of said PCR buffer solution at said plurality of positions (21, 22) is set in dependence on characteristics, in particular in dependence on a diffusion coefficient, of a nucleic acid sequence to be replicated.

6. The method of any one of the claims 1-4, wherein said nucleic acid sequences are ssDNA or ssRNA sequences comprising random sequences comprising respectively at their 5' and 3' ends fixed sequences for PCR amplification, and wherein said PCR solution is a mutagenic PCR solution and additionally comprises a plurality of target sequences immobilised on a carrier.

7. The method of claim 6,
wherein a relation between said first primer and said second primer is between 1:10 and 1:30.

8. The method of claim 6 or claim 7,
wherein said heating power is supplied such that a velocity of said PCR buffer solution at at least some positions (102, 103) of said plurality of positions (101-104) is set in dependence on a diffusion coefficient of said complex between the nucleic acid sequence and the specific target sequence immobilised on said carrier.

9. The method of any one of the preceding claims,
wherein said PCR buffer solution comprises an intercalating dye.

10. The method of any one of the preceding claims,
wherein said vessel (2) has open ends and said method comprises establishing a net flow (120) of said PCR buffer solution through said vessel (2).

11. An apparatus for amplifying one or more target nucleic acid sequence(s) in a PCR buffer solution comprising one or more target nucleic acid sequence(s), a nucleic acid polymerase, NTPs, at least a first primer and a second primer, wherein said PCR buffer solution is contained in an elongated vessel (2), said apparatus comprising:

support means (4; 61) for supporting said vessel (2);
power supply means (12-14; 12, 71-73) configured to locally supply heating power to said PCR buffer solution at a plurality of positions (21, 22; 101-104) disposed along a longitudinal direction of said vessel (2) in a time-varying manner to drive said PCR buffer solution so as to establish a fluid flow (31-34; 114, 117) circulating within said vessel (2) and so as to establish a temperature gradient within said vessel (2);
temperature adjustment means (18; 63, 78, 79) configured to adjust at least an offset temperature of said PCR buffer solution, such that

- in a first region (35, 37) within said vessel (2), said PCR buffer solution has a temperature at which a denaturation step of a PCR takes place,
- in a second region (36, 38) within said vessel (2), said PCR buffer solution has a temperature at which an annealing step and an elongation step of a PCR takes place, and
- said first region (35, 37) and said second region (36, 38) are offset relative to each other in a transverse direction of said vessel (2);

wherein said power supply means (12-14; 12, 71-73) is configured to locally supply heating power to said PCR buffer solution such that at least a fraction of said solution cyclically travels between said first region (35, 37) and said second region (36, 38).

12. The apparatus of claim 11, comprising
control means (17; 74, 75) configured to control at least one of heating power respectively supplied by said power supply means (12-14; 12, 71-73) at said plurality of positions (21, 22; 101-104) and a frequency at which said power supply means supplies heating power at said plurality of positions (21, 22; 101-104).

13. The apparatus of claim 12,
wherein said control means (17; 74, 75) is configured to control said power supply means (12-14; 12, 71-73) such that a velocity of said PCR buffer solution at at least some positions of said plurality of positions (21, 22) is set in dependence on characteristics, in particular based on a diffusion coefficient, of a nucleic acid sequence to be replicated.

14. The apparatus of claim 12,
wherein said nucleic acid sequences are ssDNA or ssRNA sequences comprising random sequences comprising respectively at their 5' and 3' ends fixed sequences for PCR amplification, and wherein said PCR solution is a mutagenic PCR solution and additionally comprises a plurality of target sequences immobilised on a carrier, and wherein said control means (17; 74, 75) is configured to control said power supply means (12-14; 12, 71-73) such that a velocity of said PCR buffer solution at at least some positions (102, 103) of said plurality of positions (101-104) is set in dependence on a diffusion coefficient of a complex between the amplified nucleic acid sequence and the specific target sequence immobilised on said carrier.

15. The apparatus of any one of claims 11-14,
    wherein said apparatus (1; 70) is configured to perform the method of any one of claims 1-10.

Fig. 1

Fig. 2

Fig. 3A

Fig. 3B

Fig. 3C

36      35      36

Primer
Annealing

Elongation

Dena-
turation

Fig. 4

Fig. 5

Fig. 6

Fig. 7

0µm/s
45µm/s

Fig. 8

93

39

Fig. 9

Fig. 10A

Fig. 10B

Fig. 11

Fig. 12

Fig. 13

Fig. 14A

Fig. 14B

Fig. 15

Europäisches
Patentamt
European
Patent Office
Office européen
des brevets

# EUROPEAN SEARCH REPORT

**Application Number**

EP 10 00 1234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 974 814 A1 (KONINKL PHILIPS ELECTRONICS NV [NL]) 1 October 2008 (2008-10-01) * paragraphs [0037] - [0040]; claims 1-11 * | 1-15 | INV. B01L3/00 B01L7/00 C12Q1/68 F04B19/00 |
| Y | US 2006/216725 A1 (LEE YOU-SEOP [KR] ET AL) 28 September 2006 (2006-09-28) * paragraphs [0003] - [0010], [0024], [0060] * | 1-15 | |
| Y,D | WEINERT FRANZ ET AL: "Optically driven fluid flow along arbitrary microscale patterns using thermoviscous expansion" JOURNAL OF APPLIED PHYSICS, AMERICAN INSTITUTE OF PHYSICS. NEW YORK, US LNKD-DOI:10.1063/1.3026526, vol. 104, no. 10, 25 November 2008 (2008-11-25), pages 104701-104701, XP012116563 ISSN: 0021-8979 * the whole document * | 1-15 | |
| X | US 2004/191830 A1 (HWANG HYUN JIN [KR] ET AL) 30 September 2004 (2004-09-30) * paragraphs [0081] - [0089]; figures 1,2 * | 1-15 | **TECHNICAL FIELDS SEARCHED (IPC)** B01L C12Q F04B |
| A | US 2002/127152 A1 (BENETT WILLIAM J [US] ET AL) 12 September 2002 (2002-09-12) * paragraphs [0008], [0011], [0026] - [0034] * | 1-15 | |

-/--

The present search report has been drawn up for all claims

| Place of search | Date of completion of the search | Examiner |
|---|---|---|
| The Hague | 14 July 2010 | Tiede, Ralph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 00 1234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EMIR VELA ET AL: "Non-contact mesoscale manipulation using laser induced convection flows" INTELLIGENT ROBOTS AND SYSTEMS, 2008. IROS 2008. IEEE/RSJ INTERNATIONAL CONFERENCE ON, IEEE, PISCATAWAY, NJ, USA, 22 September 2008 (2008-09-22), pages 913-918, XP031348450 ISBN: 978-1-4244-2057-5 * the whole document * ----- | 1-15 | |
| A | AMAR S BASU ET AL: "Virtual microfluidic traps, filters, channels and pumps using Marangoni flows" JOURNAL OF MICROMECHANICS & MICROENGINEERING, INSTITUTE OF PHYSICS PUBLISHING, BRISTOL, GB LNKD-DOI:10.1088/0960-1317/18/11/115031, vol. 18, no. 11, 1 November 2008 (2008-11-01), page 115031, XP020145049 ISSN: 0960-1317 * the whole document * ----- | 1-15 | TECHNICAL FIELDS SEARCHED (IPC) |

The present search report has been drawn up for all claims

| Place of search | Date of completion of the search | Examiner |
|---|---|---|
| The Hague | 14 July 2010 | Tiede, Ralph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 00 1234

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2010

| Patent document cited in search report | | | Publication date | Patent family member(s) | | | Publication date |
|---|---|---|---|---|---|---|---|
| EP 1974814 | A1 | | 01-10-2008 | NONE | | | |
| US 2006216725 | A1 | | 28-09-2006 | KR | 20060025027 | A | 20-03-2006 |
| US 2004191830 | A1 | | 30-09-2004 | WO | 03038127 | A1 | 08-05-2003 |
| | | | | US | 2010086975 | A1 | 08-04-2010 |
| US 2002127152 | A1 | | 12-09-2002 | WO | 02072267 | A1 | 19-09-2002 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683202 A
- US 4683195 A
- US 6428987 B2
- WO 2009141390 A1

### Non-patent literature cited in the description

- *Modern Physics Letters B,* 2004, vol. 18 (16), 775-784
- *Science,* 2002, vol. 298 (5594), 793
- **D. BRAUN ; N. L. GODDARD ; A. LIBCHABER.** Exponential DNA Replication by Laminar Convection. *PRL,* 2003, vol. 91, 138103
- **M. KRISHNAN ; V. M. UGAZ ; M. A. BURNS.** PCR in a Rayleigh-Bénard Convection Cell. *Science,* 2002, vol. 298, 793
- **P. BAASKE et al.** Extreme accumulation of nucleotides in simulated hydrothermal pore systems. *PNAS,* 2007, vol. 104 (22), 9346
- **F. M. WEINERT ; D. BRAUN.** An Optical Conveyor for Molecules. *Nano Lett.,* 2009, vol. 9 (12), 4264-4267
- **F. M. WEINERT ; D. BRAUN.** Optically driven fluid flow along arbitrary microscale patterns using thermoviscous expansion. *J. Appl. Phys.,* 2008, vol. 104, 104701
- **CORNEL MÜLHARDT.** Der Experimentator: Molekularbiologie/Genomics. Spektrum Akademischer Verlag, 2008